# EUROPEAN PATENT APPLICATION

(11) **EP 1 870 448 A1**
(43) Date of publication of application: **26.12.2007**
(21) Application number: 07110895.5
(22) Date of filing: 22.06.2007
(51) Int. Cl.: C12N 1/00, C12P 7/00

(54) **Solar power from sea**

(30) Priority: 23.06.2006 NL 1032052
(71) Applicant: Kreuger, Frederik, 2628 AK Delft (NL)
(72) Inventor: Kreuger, Frederik, 2628 AK Delft (NL)
(74) Representative: Ringeling, Patricia Louise

(57) **Abstract**

A longitudinal section of a water surface, for instance a part of the sea, where plants grow under the influence of sunlight and float between a sowing unit and a harvesting unit. The harvested plants are converted into a solid, gaseous or liquid fuel, like oil or alcohol. This section of the sea with its related ships is preferably situated in the tropics or in the sub-tropics and at the same time in areas where little or no shipping and little and no storms are to be expected.

## Description

### Technical Field

The present invention relates to the production of energy. In particular, it relates to the conversion of solar energy into fuel.

### Background Art

The most straightforward way of converting solar power into useful energy is by way of photovoltaic cells. This method, however, has two major disadvantages: it is expensive in spite of many years of research in this field and there are no ways to store the collected electric energy in sufficiently large quantities.

A better method is to grow plants in large fields, harvesting them and converting them into fuels, such as bio-diesel and bio-alcohol. This method has, nevertheless, major disadvantages as well: (a) sowing, growing, harvesting and transporting the plants is laborious, energy-consuming and expensive; (b) an even greater disadvantage is that the method occupies agricultural areas that are normally used for growing human food. For instance, the use of the Brazilian sugar fields for making bio-alcohol has raised the world sugar prize. And (c) the method makes use of the scarcest commodity in this world, fresh water.

At present there exists no method for converting solar power into chemical energy which avoids these disadvantages.

### Disclosure of Invention

Detailed description of the invention

The present invention relates to an arrangement for converting solar energy into fuel, which arrangement comprises:
(a) a sowing unit which sows plants on a water surface,
(b) a field in the water surface where the plants float and grow under the influence of sunlight, and
(c) a harvesting unit which harvests the plants for conversion into fuel.

The great advantage of the arrangement according to the present invention is that it allows for a continuous production process of fuel, which process is cheaper than state-of-the-art fuel production processes. Therefore, it allows for the production of large quantities of fuel. Another advantage is that it does not require the occupation of agricultural areas and it does not consume precious resources of fresh water.

The plants which are used to collect solar energy are plants that convert sunshine into the chemical energy of starch, fat and other biological building materials of plants. Preferably water-plants are used, more preferably algae or seaweed.

The plants may be converted into any useful fuel, be it a liquid fuel, such as oil or an alcohol; a solid fuel such as for example a combustible powder, granules or massive bits; or a gas, such as a combustible gas.

The water surface on which the plants are grown is preferably a large surface in order to allow for large-scale production. Preferably, the water-surface is a lake or a sea. The water surface is preferably in a location where a high intensity of sunshine occurs, for example in the tropics or sub-tropics. In addition, the water surface is preferably located in an area where there is little traffic of ships and only small chances of disturbing weather conditions, such as storms.

The floating plants form a field. At one side of the field new plants are added and at the other side the plants that have grown in this field are harvested and are turned into a caloric fuel like oil, alcohol or gas. The floating motion may be caused by pumping at one side and sucking at the other side of the field, but may also be caused by natural circumstances, such as natural sea currents, a suitable example is the Gulf stream. To one or more sides of the field floating borders may be applied to prevent the plants from expanding sidewards.

In one embodiment, a water surface of 100 to 1000 kilometers is exploited to produce 1000 ton of fuel a day. It is assumed that an efficiency of some tenths of a percent, for example 0.2%, may be reached in converting solar energy into chemical energy.

The plants are sown using a sowing unit, which spreads plants over the water. Preferably, the plants are spread over the water surface substantially uniformly. The sowing unit may be a floating unit, for example a ship. If several ships are used at a time, a large surface of the water may be exploited. Therefore, in one embodiment, the sowing unit comprises several ships. Microorganisms like bacteria may be added from which it is known that they stimulate the growth of plants. Other ways of fertilization may be used as well. For example, the fertilizers may be obtained from the water site itself, such as from lower layers of the water site.

The harvesting unit which is used for harvesting the plants may be a floating unit, for example a ship. The harvesting unit may move along the field to collect the plants. Alternatively, the plants may be harvested from several places at the same time. As a consequence, more plants can be harvested in the same time. Therefore, in one embodiment, the harvesting unit comprises several ships.

The harvesting unit may further comprise sucking means to suck the plants into the unit to make fuel out of it. In one embodiment, the harvesting unit has floating outriggers which are used to bring suction pipes many kilometers outwards.

In one embodiment, the harvesting unit also comprises a production unit for converting the plants into fuel. In the context of the present invention, such harvesting unit is called a factory-ship if the harvesting unit is a ship. In one embodiment, a factory-ship has a sloop or longboat attached to it as a sowing unit.

The sowing unit and the harvesting unit may be on the same side, on opposite sides or at adjacent sides of the field of plants. In one embodiment, they are at opposite sides and the plants float from one side to the other by a natural current.

In a second aspect, the present invention relates to a method for converting solar energy into fuel. The method comprises (i) sowing plants on a water-surface using a sowing unit in order to obtain a field in the water-surface where the plants float and grow by the effect of sunlight; (ii) harvesting the plants using a harvesting unit and (iii) converting the plants into fuel.

An arrangement according to the present invention, as set out above, may be used in the method of the present invention.

The plants which are sown may be in any suitable form, such as in the form of seeds, plant parts or complete plants. The grown and floating plants may extend to deeper layers of the water site. In this way the plants may form a layer or tapestry of several meters in thickness.

When the plants are sown, growth-stimulators or fertilizers may be added to promote growth. In one embodiment, growth-stimulating bacteria are added to the plants. In another embodiment, the fertilizers are obtained from the water site itself, such as from lower layers of the water site.

The plants are harvested by the harvesting unit. This may be done by any suitable means. In one embodiment, the plants are sucked into the harvesting unit. This may be accomplished by any suitable means in the art, for example by using floating outriggers which spread sucking mouths and collecting pipes over a width of several kilometers.

In one embodiment of the present invention, the plants float from one side, the side of the sowing unit, towards the opposite side, the side of the harvesting unit. The floating may be a natural process or may be forced, for example by pumping and sucking. In one embodiment, a natural sea current is used to propel the plants in the direction of the length of the field.

In another embodiment, the plants in the filed do not float from the sowing unit towards the harvesting unit, but float substantially in one place, while the sowing unit and the harvesting unit move along the edges of the field, step-wise opposed to the above supposed sea current.

Also covered by the present invention, is the embodiment wherein the harvesting unit is also used to convert the plants into a fuel. The fuel which is produced, is stored in the harvesting unit and regularly taken away by tankers. In one embodiment, part of the fuel produced is used for running the fuel production process.

The skilled person will understand that the plants may be replaced by other photosynthetic organisms, such as for example photosynthetic bacteria. The bacteria may be at the water surface or beneath it.

In a third aspect, the present invention relates to fuel obtainable by the method of the present invention.

## Claims

1. An arrangement for converting solar energy into fuel, which arrangement comprises:
(a) a sowing unit which sows plants on a water surface,
(b) a field in the water surface where the plants float and grow under the influence of sunlight, and
(c) a harvesting unit which harvests the plants for conversion into fuel.

2. An arrangement according to claim 1, wherein the plants are water-plants, preferably algae or seaweed.

3. An arrangement according to claim 1 or 2, wherein the water surface is the sea.

4. An arrangement according to claims 1-3, wherein the sowing unit or the harvesting unit is a floating unit, preferably one which comprises one or more ships.

5. An arrangement according to claims 1-4, wherein the sowing unit is a sloop or longboat of the harvesting unit.

6. A method for converting solar energy into fuel, which method comprises:
(i) sowing plants on a water-surface using a sowing unit in order to obtain a field in the water-surface where the plants float and grow by the effect of sunlight;
(ii) harvesting the plants using a harvesting unit and
(iii) converting the plants into fuel.

7. A method according to claim 6, wherein the plants are sown together with growth-stimulators, preferably growth-stimulating bacteria.

8. A method according to claim 6 or 7, wherein the sowing unit or the harvesting unit comprises one or more ships.

9. A method according to claim 6-8, wherein the harvesting unit also converts the plants into fuel.

10. Fuel obtainable by a method according to claims 6-9.
